# EUROPÄISCHE PATENTANMELDUNG

(11) **EP 4 616 783 A1**
(43) Veröffentlichungstag der Anmeldung: **17.09.2025**
(21) Anmeldenummer: 25159666.4
(22) Anmeldetag: 24.02.2025
(51) Int. Cl.: A61B 1/00, A61B 1/24, A61B 5/00, A61B 6/51, A61B 90/16, A61B 1/06, A61B 6/04

(54) **VORRICHTUNG ZUR DATENAUFNAHME**

(30) Priorität: 12.03.2024 DE 102024106996
(71) Anmelder: Dürr Dental SE, 74321 Bietigheim-Bissingen (DE)
(72) Erfinder: BLECHER, Wolf, 69502 Hemsbach (DE)
(74) Vertreter: Frenkel, Matthias Alexander

(57) **Zusammenfassung**

Die Erfindung bezieht sich auf eine Vorrichtung zur Datenaufnahme.

## Beschreibung

Die vorliegende Erfindung betrifft allgemein eine Vorrichtung zur Datenaufnahme. Im Speziellen betrifft die Erfindung eine Aufbissvorrichtung, ein medizinisches bildgebendes Gerät mit einer solchen Aufbissvorrichtung und ein Verfahren zur Röntgenaufnahme eines abzubildenden Bereichs einer Mundhöhle eines Patienten.

Vorrichtungen und Verfahren zur Datenaufnahme sind in zahlreichen Anwendungsgebieten von Bedeutung. Rein beispielhaft und ohne hierauf beschränkt zu sein, seien hier die Fahrzeugdiagnostik, Abwassertechnik, Sprengstoffprüfung, polizeiliche Einsätze und der medizinische Bereich genannt. Im medizinischen Bereich wiederum können Vorrichtungen und Verfahren zur Datenaufnahme beispielsweise für invasive medizinische Eingriffe, im Hals-Nasen-Ohren (HNO) -Bereich und im Dentalbereich Verwendung finden.

Bei medizinischen bildgebenden Systemen für die Dentaltechnik ist die Patientenpositionierung von besonderer Bedeutung. Ferner wird bei solchen medizinischen bildgebenden Systemen die Auswahl möglichst kleiner Volumen für die Bildaufnahme angestrebt. Unter anderem kann hierdurch eine Strahlenbelastung reduziert werden.

Aus dem Stand der Technik sind Verfahren bekannt, die mit einer oder zwei Kameras, oder mit Hilfe von Lichtvisieren versuchen, einem Bediener die Positionierung des Patienten in einem Digitalen Volumentomographie (DVT)-Gerät zu erleichtern. Alle diese Verfahren referenzieren die auch dem Bediener sichtbaren Merkmale des Patienten. Zusätzlich wird versucht, durch Überlagerung auf der Gesichtshaut des Patienten, Einblenden zusätzlicher Linien oder Einfärbung eines Zahnschemas auf dem Bedienfeld, dem Bediener ein Gefühl dafür zu vermitteln, welche dentale Region in der anzufertigenden DVT-Aufnahme abgebildet sein wird. Da diese Positionierung nicht zuverlässig reproduzierbar ist, wird der Bediener im Zweifelsfall eher ein größeres Volumen abbilden und somit dem Patienten zusätzliche Strahlendosis applizieren.

Die DE 10 2018 212 389 A1 betrifft eine Bestimmung einer anatomischen Position aus mit einer optischen Kamera einer Röntgeneinrichtung aufgenommenen Bilddaten. Die optische Kamera kann dabei an einer Aufnahmeanordnung und somit mit der Aufnahmeanordnung bewegbar oder ortsfest an der Röntgeneinrichtung angeordnet sein. Die von der Kamera erzeugten Bilddaten zeigen den Patienten oder einen Teil des Patienten. Die Bilddaten können dabei auch die Position der Aufnahmeanordnung zeigen und insbesondere Echtzeitbilddaten sein.

Die EP 4 140 412 A1 bezieht sich auf Verfahren zur digitalen Volumen Tomographie Aufnahme im dentalen Bereich. Gemäß einem Verfahren wird zunächst ein Patient im Röntgengerät positioniert und anschließend wird eine 3D Scoutaufnahme mit reduzierter Dosis und mit einer der vordefinierten Volumengrößen und einem vordefinierten Drehzentrum erstellt. In einem nächsten Schritt markiert ein Behandler auf der 3D Scoutaufnahme den Zahn, für den er hochauflösende Informationen haben möchte, mit einer umhüllenden Geometrie. Gemäß dem skizzierten Vorgehen wird somit versucht, das Problem der zuverlässigen Anwahl eines minimalen Volumens durch konkrete Anwahl eines Zahns und eine entsprechende Errechnung einer Abbildung auf einem Sensor bei bekannter Umlaufbahn zu lösen. Allerdings wird hierfür eine Scoutaufnahme vorausgesetzt, die für die Erstellung bereits zusätzliche Dosis beim Patienten deponiert, so dass die Dosis-Einsparung durch das kleine Volumen wieder aufgehoben oder zumindest verringert wird.

Allgemein besteht ein Bedarf nach einem verbesserten Ansatz für eine Datenaufnahme zumindest eines Teils eines Hohlraums. Im Speziellen besteht ein Bedarf nach einem verbesserten Ansatz für eine zuverlässige Patientenpositionierung und/oder eine verbesserte Auswahl eines kleinen Volumens für eine dentale Volumenaufnahme, insbesondere eine dentale DVT.

Gemäß einem ersten Aspekt der Erfindung wird eine Vorrichtung vorgeschlagen. Die Vorrichtung weist eine Anordnung und mindestens eine Aufnahmevorrichtung auf. Die Anordnung ist derart anordenbar oder aufnehmbar, dass zumindest ein Endabschnitt der Anordnung in einen Hohlraum hineinragt. Anders ausgedrückt, die Anordnung kann ausgebildet sein, derart angeordnet oder aufgenommen zu werden, dass zumindest ein Endabschnitt der Anordnung in einen Hohlraum hineinragt. Zumindest ein Abschnitt der Anordnung ist druckbeaufschlagt, wenn die Anordnung derart angeordnet oder aufgenommen ist, dass zumindest der Endabschnitt der Anordnung in den Hohlraum hineinragt. Die mindestens eine Aufnahmevorrichtung ist derart ausgebildet und an der Anordnung angeordnet, mindestens eine Datenaufnahme zumindest eines Teils des Hohlraums durchzuführen.

Anders ausgedrückt, die Vorrichtung, insbesondere die Anordnung, kann ausgebildet sein, zumindest einen Endabschnitt aufzuweisen, der derart ausgebildet ist oder sich derart erstreckt, dass er in einen Hohlraum hineinragen kann, wenn zumindest ein Abschnitt der Anordnung druckbeaufschlagt ist/wird. Die Druckbeaufschlagung kann dabei insbesondere durch die derartige Anordnung oder Aufnahme der Anordnung entstehen, dass zumindest der Endabschnitt der Anordnung in den Hohlraum hineinragt. Die Vorrichtung, insbesondere die Anordnung, kann an verschiedenen Objekten aufgenommen werden. Ist die Vorrichtung, insbesondere die Anordnung, an einem Objekt angeordnet, kann die Aufnahmevorrichtung mindestens eine Datenaufnahme zumindest eines Teils eines Hohlraums eines Objekts durchführen.

Die Vorrichtung kann in verschiedenen Bereichen zur Datenaufnahme eingesetzt werden. Rein beispielhaft und ohne hierauf beschränkt zu sein, seien hier die Fahrzeugdiagnostik, Abwassertechnik, Sprengstoffprüfung, polizeiliche Einsätze und der medizinische Bereich genannt. Im medizinischen Bereich wiederum können Vorrichtungen zur Datenaufnahme beispielsweise für invasive medizinische Eingriffe, im Hals-Nasen-Ohren (HNO) -Bereich und im Dentalbereich Verwendung finden.

Die Vorrichtung kann beispielsweise eine Aufbissvorrichtung oder eine andere geeignete Haltestruktur aufweisen oder als Aufbissvorrichtung oder als andere geeignete Haltestruktur ausgebildet sein. Die Vorrichtung kann alternativ ein Endoskop, einen Katheter oder eine andere, in eine Körperöffnung einführbare medizinische Vorrichtung aufweisen oder als solche ausgebildet sein. Die Vorrichtung kann ein nicht-medizinisches Endoskop aufweisen oder als ein solches ausgebildet sein.

Der Hohlraum kann eine Körperöffnung, wie eine Mundhöhle, eine oder mehrere Arterien oder Herzkammern aufweisen, oder als eine Körperöffnung ausgebildet sein. Alternativ kann der Hohlraum einen Raum (z. B. einen Wohnraum), einen Kessel, einen Tank, ein Silo, einen Hohlraum in Bauwerken oder Maschinen (z. B. eine U-Boot-Rohrverlegung) aufweisen oder als ein solcher Hohlraum ausgebildet sein. Die Datenaufnahme kann die Aufnahme von Bildinformation, Photonen oder Pixeln aufweisen oder als eine solche ausgebildet sein. Die Druckbeaufschlagung kann erzielt werden durch Aufnahme der Anordnung in einem Objekt. Beispielsweise kann die Druckbeaufschlagung erfolgen durch Aufnahme der Vorrichtung, insbesondere der Anordnung, zwischen Zähnen, an Ansaugknöpfen, an positionierbaren Endoskop-Halterungen, an oder unter einer Tür oder an anderen Aufnahmekomponenten.

Die mindestens eine Aufnahmevorrichtung kann an dem Endabschnitt der Anordnung ausgebildet sein. Auf diese Weise kann die Aufnahmevorrichtung auf einfache Weise eine Datenaufnahme durchführen, wenn zumindest der Endabschnitt in den Hohlraum hineinragt.

Der Endabschnitt der Anordnung ist beispielsweise derart ausgebildet, dass er nicht druckbeaufschlagt ist, wenn die Anordnung derart angeordnet oder aufgenommen ist, dass zumindest der Endabschnitt der Anordnung in den Hohlraum hineinragt. Auf diese Weise wird zwar zumindest ein Abschnitt, beispielsweise der aufgenommene Abschnitt, druckbeaufschlagt, der Endabschnitt jedoch nicht. Dadurch kann der Endabschnitt auf verlässliche Weise in den Hohlraum hineinragen. Dies ist insbesondere auch dann von Vorteil, wenn an dem Endabschnitt die Aufnahmevorrichtung angeordnet ist.

Die Vorrichtung kann ferner mindestens eine ausstrahlende Vorrichtung aufweisen. Die ausstrahlende Vorrichtung kann an der Anordnung angeordnet sein. Die ausstrahlende Vorrichtung kann eine Lichtquelle, eine Röntgenquelle, eine Laserquelle, eine Infrarot-Quelle, Ultraviolett(UV)-Quelle, eine Terahertzwellen-Quelle und/oder eine Hochfrequenz(HF)-Puls-Quelle aufweisen oder als eine Lichtquelle, eine Röntgenquelle, eine Laserquelle, eine Infrarot-Quelle, Ultraviolett(UV)-Quelle, eine Terahertzwellen-Quelle oder eine Hochfrequenz(HF)-Puls-Quelle ausgebildet sein.

Die mindestens eine ausstrahlende Vorrichtung kann an dem Endabschnitt der Anordnung ausgebildet sein. Beispielsweise können die Aufnahmevorrichtung und die ausstrahlende Vorrichtung an dem Endabschnitt der Anordnung ausgebildet sein. Auf diese Weise können die Aufnahmevorrichtung und die ausstrahlende Vorrichtung gemeinsam zumindest den Teil des Hohlraums beleuchten und/oder bestrahlen, wenn zumindest der Endabschnitt in den Hohlraum hineinragt.

Die ausstrahlende Vorrichtung kann derart ausgebildet und an der Anordnung angeordnet sein, im Zusammenwirken mit der Aufnahmevorrichtung zumindest einen Teil des Hohlraums abzubilden. Beispielsweise kann eine Datenaufnahme zumindest eines Teils des Hohlraums durch Zusammenwirken der Aufnahmevorrichtung und der ausstrahlenden Vorrichtung erfolgen.

Die ausstrahlende Vorrichtung kann mindestens eine Beleuchtungskomponente aufweisen oder als mindestens eine Beleuchtungskomponente ausgebildet sein. Die Beleuchtungskomponente kann derart ausgebildet und an der Anordnung angeordnet sein, zumindest einen Teil einer Mundhöhle eines Patienten auszuleuchten. Die mindestens eine Beleuchtungskomponente kann eine Lichtquelle, z. B. eine Leuchtdiode (LED), aufweisen oder als Lichtquelle, z. B. als LED, ausgebildet sein.

Die Vorrichtung kann ausgebildet sein, die mindestens eine Datenaufnahme an ein übergeordnetes System zur weiteren Verwendung weiterzuleiten. Das übergeordnete System kann die Datenaufnahme zur Einflussnahme auf ein unabhängiges System nutzen. Das unabhängige System kann von der Vorrichtung und/oder dem übergeordneten System unabhängig sein. Das unabhängige System kann zumindest einen Teil eines menschlichen Körpers oder ein medizinisches bildgebendes System aufweisen. Beispielsweise kann die Datenaufnahme zur Ausrichtung zumindest eines Teils des menschlichen Körpers oder zur Gewinnung von Informationen über zumindest einen Teil des menschlichen Körpers, beispielsweise ein Krebsgeschwür, verwendet werden.

Gemäß einem zweiten Aspekt wird eine Aufbissvorrichtung für ein medizinisches bildgebendes Gerät, insbesondere ein dentales Röntgengerät, vorgeschlagen. Die Aufbissvorrichtung weist eine Aufbissanordnung und mindestens eine Kamera auf. Die Aufbissanordnung ist derart zwischen zumindest einem Zahn eines Oberkiefers und zumindest einem Zahn eines Unterkiefers eines Patienten anordenbar oder aufnehmbar, dass zumindest ein Endabschnitt der Aufbissanordnung in eine Mundhöhle des Patienten hineinragt. Anders ausgedrückt, die Aufbissanordnung ist ausgebildet, zwischen zumindest einem Zahn eines Oberkiefers und zumindest einem Zahn eines Unterkiefers eines Patienten derart aufgenommen zu werden, dass zumindest ein Endabschnitt der Aufbissanordnung in eine Mundhöhle des Patienten hineinragt. Die mindestens eine Kamera ist derart ausgebildet und an der Aufbissanordnung angeordnet, mindestens eine Bildaufnahme zumindest eines Teils der Mundhöhle des Patienten aufzunehmen / zu erstellen / zu erzeugen.

Die mindestens eine Bildaufnahme kann als mindestens eine optische Aufnahme bezeichnet werden. Die mindestens eine Kamera kann als mindestens eine optische Kamera bezeichnet werden. Der mindestens eine Teil der Mundhöhle kann einen oder mehrere Zähne aufweisen oder als einer oder mehrere Zähne ausgebildet sein.

Die mindestens eine Kamera kann an dem Endabschnitt der Aufbissanordnung ausgebildet sein. Der Endabschnitt kann bei Aufnahme der Aufbissanordnung zwischen den Zähnen des Patienten in die Mundhöhle des Patienten ragen. Der Endabschnitt kann beispielsweise zylinderförmig ausgebildet sein. Der Endabschnitt kann einen kreisförmigen oder rechteckigen Querschnitt aufweisen. Eine beispielsweise flache Endseite oder Endfläche des beispielsweise zylinderförmigen Endabschnitts kann die mindestens eine Kamera aufweisen. Der Endabschnitt kann, je nach Ansicht, T-förmig oder U-förmig oder V-förmig ausgebildet sein. Beide beispielsweise flache Endseiten oder Endflächen des beispielsweise T-förmigen oder U-förmigen oder V-förmigen Endabschnitts können jeweils eine der mindestens eine Kamera aufweisen. Zusätzlich oder alternativ können eine oder mehrere Kameras der mindestens einen Kamera an einem Bogen oder Schenkel eines beispielsweise U-förmigen oder V-förmigen Endabschnitts angeordnet sein.

Die Aufbissvorrichtung kann ferner mindestens eine Beleuchtungskomponente aufweisen. Die mindestens eine Beleuchtungskomponente kann derart ausgebildet und an der Aufbissanordnung angeordnet sein, zumindest einen Teil der Mundhöhle des Patienten auszuleuchten. Der von der mindestens einen Beleuchtungskomponente ausgeleuchtete Teil der Mundhöhle des Patienten kann zumindest nahezu dem von der mindestens einen Kamera aufgenommenen Teil der Mundhöhle des Patienten entsprechen.

Die mindestens eine Beleuchtungskomponente kann an dem Endabschnitt der Aufbissanordnung ausgebildet sein. Der Endabschnitt kann beispielsweise zylinderförmig ausgebildet sein. Der Endabschnitt kann einen kreisförmigen oder rechteckigen Querschnitt aufweisen. Eine beispielsweise flache Endseite oder Endfläche des beispielsweise zylinderförmigen Endabschnitts kann die mindestens eine Beleuchtungskomponente aufweisen. Die mindestens eine Kamera und die mindestens eine Beleuchtungskomponenten können beispielsweise gemeinsam an der gleichen Endseite oder Endfläche angeordnet sein. Die mindestens eine Beleuchtungskomponente kann die mindestens eine Kamera umgeben oder umgekehrt. Der Endabschnitt kann T-förmig oder U-förmig ausgebildet sein. Beide beispielsweise flache Endseiten oder Endflächen des beispielsweise T-förmigen oder U-förmigen Endabschnitts können jeweils eine der mindestens einen Kamera aufweisen und die mindestens eine Beleuchtungskomponente kann getrennt / separiert von der mindestens einen Kamera an einer anderen Stelle der Aufbissanordnung angeordnet sein. Die mindestens eine Beleuchtungskomponente kann eine oder mehrere Leuchtdioden (LEDs) aufweisen oder als eine oder mehrere LEDs ausgebildet sein.

Die mindestens eine Beleuchtungskomponente kann getrennt von der mindestens einen Kamera an der Aufbissvorrichtung, insbesondere dem Endabschnitt der Aufbissvorrichtung, angeordnet sein. Alternativ kann die mindestens eine Beleuchtungskomponente gemeinsam mit der mindestens einen Kamera an der Aufbissvorrichtung, insbesondere dem Endabschnitt der Aufbissvorrichtung, angeordnet sein. Beispielsweise kann die Beleuchtungskomponente, insbesondere als eine LED oder LED-Anordnung, die mindestens eine Kamera beispielsweise kreisrund umgeben oder umgekehrt.

Gemäß einem dritten Aspekt wird ein medizinisches bildgebendes Gerät vorgeschlagen. Das medizinische bildgebende Gerät kann eine Aufbissvorrichtung gemäß dem zweiten Aspekt und eine Röntgenaufnahmevorrichtung aufweisen. Die Aufbissvorrichtung gemäß dem zweiten Aspekt weist eine Aufbissanordnung und mindestens eine Kamera auf. Die Aufbissanordnung ist zwischen zumindest einem Zahn eines Oberkiefers und zumindest einem Zahn eines Unterkiefers eines Patienten derart anordenbar oder aufnehmbar, dass zumindest ein Endabschnitt der Aufbissanordnung in eine Mundhöhle eines Patienten hineinragt. Anders ausgedrückt, die Aufbissanordnung ist ausgebildet, zwischen zumindest einem Zahn eines Oberkiefers und zumindest einem Zahn eines Unterkiefers eines Patienten derart aufgenommen zu werden, dass zumindest ein Endabschnitt der Aufbissanordnung in eine Mundhöhle eines Patienten hineinragt. Die mindestens eine Kamera ist derart ausgebildet und an der Aufbissanordnung angeordnet, mindestens eine Bildaufnahme / optische Aufnahme zumindest eines Teils der Mundhöhle des Patienten aufzunehmen / zu erstellen / zu erzeugen. Die Röntgenaufnahmevorrichtung ist ausgebildet, mindestens eine Röntgenaufnahme eines abzubildenden Bereichs der Mundhöhle des Patienten unter Berücksichtigung von Informationen über die Bildaufnahme zumindest des Teils der Mundhöhle des Patienten durchzuführen. Anders ausgedrückt, bei der Durchführung / Erstellung einer Röntgenaufnahme des abzubildenden Bereichs der Mundhöhle des Patienten können die Informationen über die Bildaufnahme zumindest des Teils der Mundhöhle des Patienten verwendet / berücksichtigt werden. Beispielsweise kann die mindestens eine Röntgenaufnahme des abzubildenden Bereichs der Mundhöhle des Patienten unter Berücksichtigung der Bildaufnahme zumindest des Teils der Mundhöhle des Patienten durchgeführt werden.

Das medizinische bildgebende Gerät kann ein dentales Röntgengerät aufweisen oder als ein dentales Röntgengerät ausgebildet sein. Das medizinische bildgebende Gerät kann insbesondere ein extraorales dentales Röntgengerät aufweisen oder als ein extraorales dentales Röntgengerät ausgebildet sein.

Der abzubildende Bereich der Röntgenaufnahme kann zumindest nahezu dem Teil der Mundhöhle entsprechen. Alternativ kann der abzubildende Bereich der Röntgenaufnahme zumindest teilweise von dem Teil der Mundhöhle abweichen.

Die mindestens eine Röntgenaufnahme des abzubildenden Bereichs kann mindestens eine hochauflösende und/oder dreidimensionale Röntgenaufnahme aufweisen oder mindestens einer hochauflösenden und/oder dreidimensionalen Röntgenaufnahme entsprechen. Die mindestens eine Röntgenaufnahme des abzubildenden Bereichs kann mindestens eine Digitale Volumentomographie (DVT) -Aufnahme aufweisen oder mindestens einer DVT Aufnahme entsprechen. Unter DVT wird für gewöhnliche ein dreidimensionales, bildgebendes Tomographie-Verfahren unter Nutzung von Röntgenstrahlen verstanden. Ähnlich wie bei der Computertomographie (CT) oder der Magnetresonanztomographie (MRT) dient auch die DVT der Erzeugung von Schnittbildern. DVT-Geräte generieren ihre Volumendatensätze mittels eines mathematischen Prozesses (Rückprojektion) aus in der Regel mehreren Hundert einzelnen Röntgen-Projektionsaufnahmen. Im Gegensatz zum zweidimensionalen Röntgen, wo die Information in Strahlengangsrichtung stark reduziert wird, ermöglicht das dreidimensionale Röntgen, wie beispielsweise die DVT, die Darstellung der abgebildeten anatomischen Strukturen in allen Raumrichtungen.

Das medizinische bildgebende Gerät kann ferner eine Recheneinheit aufweisen. Die Recheneinheit kann ausgebildet sein, den abzubildenden Bereich der Mundhöhle des Patienten unter Berücksichtigung von Informationen über die Bildaufnahme zumindest des Teils der Mundhöhle des Patienten zu ermitteln. Die Recheneinheit kann ausgebildet sein, den abzubildenden Bereich der Mundhöhle des Patienten unter Berücksichtigung der Bildaufnahme zumindest des Teils der Mundhöhle des Patienten zu ermitteln. Die Recheneinheit kann beispielsweise den abzubildenden Bereich der Mundhöhle des Patienten beispielsweise automatisch unter Berücksichtigung von Informationen über die Bildaufnahme zumindest des Teils der Mundhöhle des Patienten ermitteln. Alternativ kann der abzubildende Bereich der Mundhöhle des Patienten beispielsweise unter Berücksichtigung von Informationen über die Bildaufnahme zumindest des Teils der Mundhöhle des Patienten durch einen Benutzer eingegeben werden. Beispielsweise kann der abzubildende Bereich auf der Bildaufnahme zumindest des Teils der Mundhöhle durch einen Benutzer ausgewählt werden.

Zusätzlich oder alternativ kann die Recheneinheit ausgebildet sein, Informationen über eine Bahnkurve des medizinischen bildgebenden Geräts basierend auf den Informationen über die Bildaufnahme zumindest des Teils der Mundhöhle des Patienten zu ermitteln. Die Recheneinheit kann ausgebildet sein, Informationen über eine Bahnkurve des medizinischen bildgebenden Geräts basierend auf der Bildaufnahme zumindest des Teils der Mundhöhle des Patienten zu ermitteln. Die Informationen über die Bahnkurve können beispielsweise ein Rotationszentrum oder mehrere Rotationszentren der Bahnkurve des medizinischen bildgebenden Geräts aufweisen. Die Informationen über die Bahnkurve können Informationen über eine Umlauf-Geschwindigkeit und/oder einen genauen Verlauf der Bahnkurve aufweisen. Die Informationen über eine Umlauf-Geschwindigkeit können Informationen über eine Umlaufgeschwindigkeit einer beispielsweise rotierenden Strahlenquelle, beispielsweise Röntgenquelle, und/oder eines Detektors, insbesondere eines Röntgen-Detektors, aufweisen.

Wie oben genannt kann das medizinische bildgebende Gerät ein extraorales Röntgengerät aufweisen oder als ein solches ausgebildet sein. Das extraorale Röntgengerät kann als ein Panorama-Röntgengerät, ein DVT-Gerät oder ein Hybrid-Gerät aus Panorama- und DVT-Gerät ausgebildet sein. Das Röntgengerät kann einen Röntgenstrahler und einen Röntgendetektor aufweisen. Der Röntgenstrahler und/oder der Röntgendetektor kann/können sich auf einer zu bestimmenden Bahnkurve bewegen, insbesondere um einen Teil, beispielsweise Kopf, eines Patienten bewegen. In dem medizinischen bildgebenden Gerät kann eine Standard-Bahnkurve hinterlegt oder abgespeichert sein, die unabhängig von patientenspezifischen Informationen abgefahren wird. Basierend auf den Informationen über die mindestens einen Bildaufnahme kann die Standard-Bahnkurve angepasst werden. Insbesondere kann ein Rotationszentrum oder können Rotationszentren der Standard-Bahnkurve angepasst werden. Ferner kann eine Blenden-Position und/oder eine Abbildungsposition auf einem Detektor, insbesondere einem Röntgen-Detektor, des medizinischen bildgebenden Geräts angepasst werden.

Die Aufbissvorrichtung, z. B. die Aufbissanordnung und/oder die mindestens eine Kamera, kann drahtlos und/oder drahtgebunden mit der Recheneinheit verbunden oder verbindbar sein, d. h. die Verbindung zwischen der Aufbissvorrichtung, z. B. der Aufbissanordnung und/oder der mindestens einen Kamera, und der Recheneinheit kann eine drahtgebundene Verbindung, eine drahtlose Verbindung oder eine Kombination aus beiden aufweisen. Über die drahtlose und/oder drahtgebundene Verbindung kann die Aufbissvorrichtung, z.B. die Aufbissanordnung und/oder die mindestens eine Kamera, die von der mindestens einen Kamera aufgenommene mindestens eine Bildaufnahme an die Recheneinheit übermitteln.

Das medizinische bildgebende Gerät kann eine Ausgabeeinheit aufweisen. Die Ausgabeeinheit kann ausgebildet sein, die Bildaufnahme zumindest eines, insbesondere des, Teils der Mundhöhle auszugeben. Insbesondere kann die Ausgabeeinheit ausgebildet sein, eine Darstellung einer Überlagerung der Bildaufnahme zumindest eines, insbesondere des, Teils der Mundhöhle und des abzubildenden Bereichs der Mundhöhle des Patienten, auszugeben.

Das medizinische bildgebende Gerät kann eine Eingabeeinheit aufweisen. Die Eingabeeinheit kann beispielsweise mit der Ausgabeeinheit kombiniert sein oder auf der Ausgabeeinheit ausgebildet sein. Beispielsweise kann die Eingabeeinheit eine oder mehrere Tasten und/oder Touchscreen-Elemente auf der Ausgabeeinheit aufweisen. Die Eingabeeinheit kann ausgebildet sein, eine Eingabe oder Auswahl einer vordefinierten Volumengröße für den abzubildenden Bereich zu erhalten. Zusätzlich oder alternativ kann die Eingabeeinheit ausgebildet sein, eine Markierung oder Auswahl des abzubildenden Bereichs auf der Bildaufnahme zumindest eines, insbesondere des, Teils der Mundhöhle des Patienten zu erhalten.

Die mindestens eine Kamera kann ausgebildet sein, mindestens eine Zwischenbildaufnahme zumindest eines, insbesondere des, Teils der Mundhöhle des Patienten während einer Durchführung der Röntgenaufnahme des abzubildenden Bereichs der Mundhöhle des Patienten aufzunehmen / zu erstellen. Die Zwischenbildaufnahme kann dazu dienen, Informationen über eine mögliche Patientenbewegung bereitzustellen oder eine mögliche Patientenbewegung festzustellen. Auf diese Weise können Bild-Artefakte reduziert, insbesondere minimiert, werden.

Gemäß einem vierten Aspekt wird ein Verfahren zur Röntgenaufnahme eines abzubildenden Bereichs einer Mundhöhle eines Patienten vorgeschlagen. Das Verfahren umfasst ein derartiges Anordnen oder Aufnehmen einer Aufbissanordnung eines medizinischen bildgebenden Geräts zwischen zumindest einem Zahn eines Oberkiefers und zumindest einem Zahn eines Unterkiefers eines Patienten, dass zumindest ein Endabschnitt der Aufbissanordnung in eine Mundhöhle eines Patienten hineinragt. Das Verfahren umfasst ein Durchführen mindestens einer Bildaufnahme zumindest eines Teils der Mundhöhle des Patienten mit mindestens einer an der Aufbissanordnung angeordneten Kamera. Das Verfahren umfasst ein Durchführen einer Röntgenaufnahme, mit einer Röntgenaufnahmevorrichtung des medizinischen bildgebenden Geräts, mindestens eines abzubildenden Bereichs der Mundhöhle des Patienten unter Berücksichtigung von Informationen über die Bildaufnahme zumindest eines, insbesondere des, Teils der Mundhöhle des Patienten.

Ein fünfter Aspekt bezieht sich auf ein Computerprogramm mit Programmcodemitteln, das, wenn es in einen Computer oder einen Prozessor (beispielsweise einen Mikroprozessor, Mikrocontroller oder digitalen Signalprozessor (DSP)) geladen ist, oder auf einem Computer oder Prozessor (z.B. einem Mikroprozessor, Mikrocontroller oder DSP) läuft, den Computer oder Prozessor (z.B. Mikroprozessor, Mikrocontroller oder DSP) dazu veranlasst, einen oder mehrere Schritte oder alle Schritte der zuvor in Bezug auf die Recheneinheit und/oder die Röntgenaufnahme beschriebenen Verfahrensschritte auszuführen. Zudem wird ein Programmspeichermedium oder Computerprogrammprodukt mit dem genannten Computerprogramm bereitgestellt.

Ferner kann beispielsweise das Computerprogramm gemäß dem fünften Aspekt in der Recheneinheit des medizinischen bildgebenden Geräts gemäß dem dritten Aspekt gespeichert sein und die Recheneinheit dazu veranlassen, einen oder mehrere oder alle der zuvor in Bezug auf die Recheneinheit beschriebenen Aspekte und/oder Schritte des Verfahrens gemäß dem vierten Aspekt auszuführen. Ferner kann beispielsweise das Computerprogramm gemäß dem fünften Aspekt in der Recheneinheit des medizinischen bildgebenden Geräts gemäß dem dritten Aspekt gespeichert sein und die Recheneinheit dazu veranlassen, einen oder mehrere oder alle der zuvor in Bezug auf die Recheneinheit beschriebenen Details, Aspekte und/oder Merkmale auszuführen.

Auch wenn einige der voranstehend beschriebenen Details in Bezug auf die Vorrichtung gemäß dem ersten Aspekt oder die Aufbissvorrichtung gemäß dem zweiten Aspekt beschrieben wurden, so können diese Aspekte auch in entsprechender Weise in dem medizinischen bildgebenden Gerät, dem Verfahren oder einem das Verfahren oder die Recheneinheit implementierenden Computerprogramm realisiert werden und umgekehrt. Genauso können die voranstehend in Bezug auf das Verfahren beschriebenen Aspekte in dem medizinischen bildgebenden Gerät, dem Verfahren oder einem das Verfahren oder die Recheneinheit implementierenden Computerprogramm realisiert werden und umgekehrt.

Die vorliegende Offenbarung soll weiter anhand von Figuren erläutert werden. Diese Figuren zeigen schematisch:
- Figur 1: einen Schädel mit einer Aufbissschiene;
- Figur 2: eine Aufbissvorrichtung gemäß einem Ausführungsbeispiel in einer Seitenansicht;
- Figur 3: eine Aufbissvorrichtung gemäß einem Ausführungsbeispiel in einer Draufsicht von oben;
- Figur 4: ein medizinisches bildgebendes Gerät mit einer Aufbissvorrichtung gemäß den Ausführungsbeispielen aus Figuren 2 und 3;
- Figur 5: eine Ausgabeeinheit, die mit einer Aufbissvorrichtung gemäß den Ausführungsbeispielen aus Figuren 2 und 3 und/oder dem medizinischen bildgebenden Gerät aus Figur 4 einsetzbar ist;
- Figur 6: ein Flussdiagramm eines Verfahrens gemäß einem Ausführungsbeispiel;

Im Folgenden werden, ohne hierauf beschränkt zu sein, spezifische Details dargelegt, um ein vollständiges Verständnis der vorliegenden Offenbarung zu liefern. Es ist einem Fachmann jedoch klar, dass die vorliegende Offenbarung in anderen Ausführungsbeispielen verwendet werden kann, die von den nachfolgend dargelegten Details abweichen können. Beispielsweise wird im Folgenden konkret eine Aufbissvorrichtung als ein Beispiel für eine Vorrichtung zur Datenaufnahme beschrieben. Andere Ausgestaltungen der Vorrichtung für andere Einsatzzwecke sind jedoch denkbar und möglich. Ferner werden im Folgenden spezifische Konfigurationen und Ausgestaltungen eines medizinischen bildgebenden Geräts und/oder einer Aufbissvorrichtung mit Aufbissanordnung beschrieben, die nicht als einschränkend anzusehen sind.

Es ist dem Fachmann klar, dass die nachfolgend dargelegten Erklärungen unter Verwendung von Hardwareschaltungen, Softwaremitteln oder einer Kombination davon implementiert sein/werden können. Die Softwaremittel können im Zusammenhang stehen mit programmierten Mikroprozessoren, einer künstlichen Intelligenz oder einem allgemeinen Computer, einer ASIC (Application Specific Integrated Circuit; auf Deutsch: anwendungsspezifische integrierte Schaltung) und/oder DSPs (Digital Signal Processors; auf Deutsch: digitale Signalprozessoren). Es ist zudem klar, dass auch dann, wenn die nachfolgenden Details in Bezug auf ein Verfahren beschrieben werden, diese Details auch in einer geeigneten Vorrichtungseinheit, einem Computerprozessor oder einem mit einem Prozessor verbundenen Speicher realisiert sein können, wobei der Speicher mit einem oder mehreren Programmen versehen ist, die das Verfahren durchführen, wenn sie durch den Prozessor ausgeführt werden.

Die Ausführungsbeispiele können realisiert sein und/oder verwendet werden in verschiedenen medizinischen bildgebenden Geräten. Rein beispielhaft seien hier Röntgengeräte, insbesondere Panorama-Röntgengeräte, DVT-Röntgengeräte oder Hybrid-Geräte mit Panorama- und DVT-Röntgengeräten, genannt.

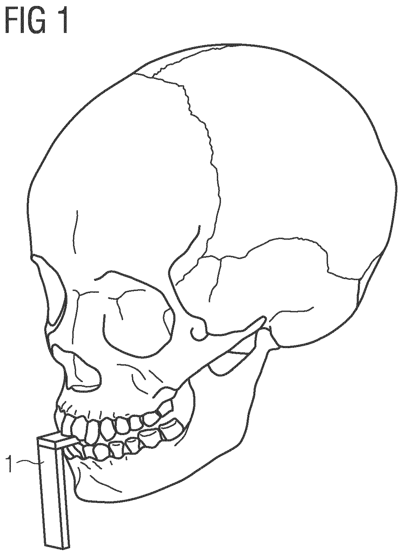

Figur 1 zeigt schematisch einen Schädel eines Patienten sowie eine herkömmliche Aufbissschiene 1. Wie zu erkennen, beißt der Patient vor und/oder während der Durchführung einer Röntgenaufnahme(n) in die Aufbissschiene 1. Über den Eingriff der Zähne in die Aufbissschiene erhofft man sich eine teilweise Fixierung des Kopfes und insbesondere, dass der Kopf des Patienten während der Röntgenaufnahme(n) möglichst ruhig und in einer festen, beispielsweise kalibrierten, Position verbleibt. Insbesondere wird durch den Aufbiss versucht, eine Translation des Schädels zu begrenzen oder zumindest nahezu zu verhindern. Auf diese Weise erhofft man sich, möglichst genaue und scharfe Röntgenaufnahmen erstellen zu können. In der Praxis zeigt sich jedoch, dass diese Vorgehensweise fehleranfällig ist. Zum einen verbleibt der Kopf für gewöhnlich nicht in der erhofften Ruhestellung. Zum anderen beruht die Positionierung auf Erfahrungswerten des Praxispersonals und unterliegt dadurch menschlichen Fehlern und Fehleinschätzungen.

Bei medizinischen bildgebenden Systemen für die Dentaltechnik ist die Patientenpositionierung von besonderer Bedeutung. Ferner wird bei solchen medizinischen bildgebenden Systemen die Auswahl möglichst kleiner Volumen für die Bildaufnahme angestrebt. Unter anderem kann hierdurch eine Strahlenbelastung reduziert werden. Die Patientenpositionierung und somit die Auswahl des abzubildenden Bereiches, ist jedoch nicht zuverlässig reproduzierbar. Daher wird der Bediener im Zweifelsfall eher ein größeres Volumen abbilden und somit dem Patienten zusätzliche Strahlendosis applizieren.

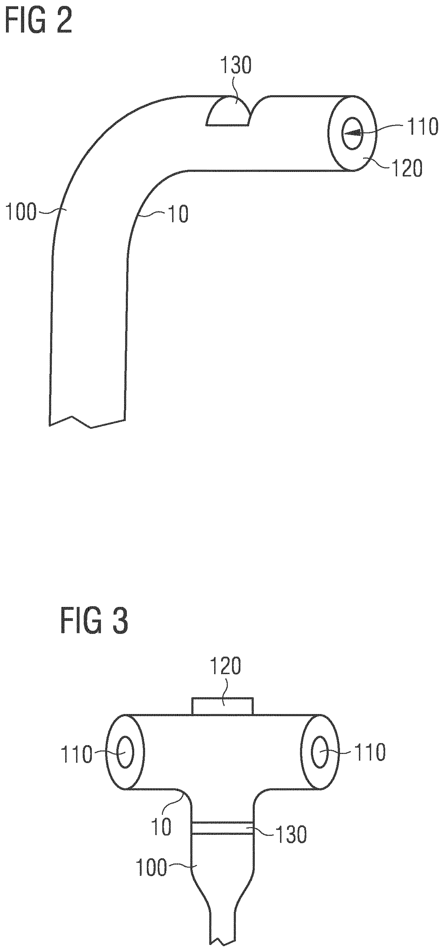

In Figur 2 ist eine Aufbissvorrichtung 10 gemäß einem Ausführungsbeispiel in einer Seitenansicht schematisch dargestellt. Die Aufbissvorrichtung 10 ist geeignet für ein medizinisches bildgebendes Gerät, insbesondere ein dentales Röntgengerät. Die Aufbissvorrichtung 10 weist eine Aufbissanordnung 100 auf. Die Aufbissanordnung 100 kann insbesondere als eine Aufbissschiene ausgebildet sein oder eine solche aufweisen. Die Aufbissanordnung 100 ist zwischen zumindest einem Zahn eines Oberkiefers und zumindest einem Zahn eines Unterkiefers eines Patienten derart anordenbar oder aufnehmbar, dass zumindest ein Endabschnitt der Aufbissanordnung 100 in eine Mundhöhle des Patienten hineinragt. Die grundsätzliche Positionierung einer Aufbissschiene 1 zwischen Zähnen eines Patienten ist in Figur 1 gezeigt und in Bezug auf Figur 1 skizziert worden.

Die Aufbissvorrichtung 10 weist ferner in dem Beispiel aus Figur 2 beispielhaft genau eine Kamera 110 auf, um zu veranschaulichen, dass mindestens eine Kamera 110 vorgesehen sein kann. Die Kamera 110 ist an der Aufbissanordnung 100 angeordnet. Die Kamera 110 ist ausgebildet und an der Aufbissanordnung 100 derart angeordnet, dass sie mindestens eine Bildaufnahme / optische Aufnahme zumindest eines Teils der Mundhöhle des Patienten aufnehmen kann.

Hierzu befindet sich die Kamera 110 an oder in einem Endabschnitt der Aufbissanordnung 100, der bei Aufnahme der Aufbissvorrichtung 10 zwischen den Zähnen des Patienten bei fachgerechter Anwendung in die Mundhöhle des Patienten ragt. Die Kamera 110 ist in dem Beispiel aus Figur 2 an einer Endfläche der Aufbissanordnung 100 ausgebildet. Genauer gesagt, befindet sich die Kamera 110 an einer Endfläche oder Grundfläche der in Figur 2 beispielhaft zumindest abschnittsweise röhrenförmig oder zylinderförmig ausgebildeten Aufbissanordnung 100.

Die Aufbissvorrichtung 10 weist ferner in dem Beispiel aus Figur 2 beispielhaft eine einzige Beleuchtungskomponente 120 auf, um zu veranschaulichen, dass mindestens eine Beleuchtungskomponente 120 vorgesehen sein kann. Die Beleuchtungskomponente 120 ist an der Aufbissanordnung 100 angeordnet. Die Beleuchtungskomponente 120 ist derart ausgebildet und an dem Endabschnitt der Aufbissanordnung 100 angeordnet, dass sie zumindest einen Teil der Mundhöhle des Patienten ausleuchten kann. Die Beleuchtungskomponente 120 kann beispielsweise als mindestens eine Leuchtdiode (LED) ausgebildet sein oder mindestens eine LED aufweisen.

Hierzu befindet sich die Beleuchtungskomponente 120 an dem Endabschnitt der Aufbissanordnung 100, der bei Aufnahme der Aufbissvorrichtung 10 zwischen den Zähnen des Patienten bei fachgerechter Anwendung in die Mundhöhle des Patienten ragt. Die Beleuchtungskomponente 120 ist in dem Beispiel aus Figur 2 an einer Endfläche der Aufbissanordnung 100 ausgebildet. Genauer gesagt, befindet sich die Beleuchtungskomponente 120 an einer Endfläche oder Grundfläche der in Figur 2 beispielhaft zumindest abschnittsweise röhrenförmig oder zylinderförmig ausgebildeten Aufbissanordnung 100. In dem Beispiel aus Figur 2 ist die Beleuchtungskomponente 120 ferner beispielhaft scheibenförmig oder kreisringförmig ausgebildet und umgibt in ihrer Scheibenform oder Kreisringform die Kamera 110 beispielhaft vollständig.

In dem Beispiel aus Figur 2 sind die Kamera 110 und die Beleuchtungskomponente 120 gemeinsam an einer gleichen Fläche, nämlich der Endfläche oder Grundfläche, der Aufbissanordnung 100 angeordnet. Dementsprechend können der Blickwinkel der Kamera 110 und die Beleuchtungsrichtung der Beleuchtungskomponente 120 zumindest nahezu übereinstimmen. Auf diese Weise kann effizient der durch die Kamera 110 aufzunehmende Bereich der Mundhöhle durch die Beleuchtungskomponente 120 ausgeleuchtet werden.

Die Aufbissvorrichtung 10 weist ferner eine Aufbisskerbe 130 auf. Die Aufbisskerbe 130 ist in der Aufbissanordnung 100, beispielsweise einer Aufbissschiene, der Aufbissvorrichtung 10 ausgebildet. Die Aufbisskerbe 130 ist in dem Beispiel aus Figur 2 beispielhaft nur an einer Oberseite der Aufbissanordnung 100 zur Aufnahme von mindestens einem Zahn des Oberkiefers eines Patienten ausgebildet. Zusätzlich oder alternativ kann eine entsprechende Kerbe an einer Unterseite der Aufbissanordnung 100 zur Aufnahme von mindestens einem Zahn des Unterkiefers eines Patienten ausgebildet sein. Die Aufbisskerbe 130 ist derart an der Aufbissanordnung 100 angeordnet oder positioniert, dass bei fachgerechter Anwendung und Aufnahme der Aufbissvorrichtung 10 zwischen den Zähnen eines Patienten, die Kamera 110 und die Beleuchtungskomponente 120 in Richtung der Mundhöhle zeigen und/oder in der Mundhöhle des Patienten aufgenommen sind, um eine Aufnahme und Ausleuchtung zumindest eines Teils der Mundhöhle des Patienten zu gewährleisten.

In Figur 3 ist eine Aufbissvorrichtung 10 gemäß einem Ausführungsbeispiel in einer Draufsicht von oben schematisch dargestellt. Die Aufbissvorrichtung 10 gemäß Figur 3 kann als eine Variante der Aufbissvorrichtung 10 aus Figur 2 angesehen werden. Daher werden bei der Beschreibung der Aufbissvorrichtung 10 aus Figur 3 nicht alle bereits in Bezug auf die Aufbissvorrichtung 10 aus Figur 2 beschriebenen Details wiederholt.

Die Aufbissvorrichtung 10 weist ferner in dem Beispiel aus Figur 3 beispielhaft zwei Kameras 110 auf, um zu veranschaulichen, dass mindestens eine Kamera 110 und insbesondere mehrere Kameras 110 vorgesehen sein können. Die Kameras 110 sind an der Aufbissanordnung 100 angeordnet. Die Kameras 110 sind ausgebildet und an der Aufbissanordnung 100 derart angeordnet, dass sie mindestens eine Bildaufnahme / optische Aufnahme zumindest eines Teils der Mundhöhle des Patienten aufnehmen können.

Hierzu befinden sich die Kameras 110 an oder in einem Endabschnitt der Aufbissanordnung 100, der bei Aufnahme der Aufbissvorrichtung 10 zwischen den Zähnen des Patienten bei fachgerechter Anwendung in die Mundhöhle des Patienten ragt. Genauer gesagt, sind die Kameras 110 in dem Beispiel aus Figur 3 jeweils an einer Endfläche der Aufbissanordnung 100 ausgebildet.

Der Endabschnitt der Aufbissanordnung 100 kann in der in Figur 3 gezeigten Draufsicht als T-förmig bezeichnet werden. In diesem Fall befinden sich die Kameras 110 jeweils an einer Endfläche oder Grundfläche des in Figur 3 beispielhaft T-förmig ausgebildeten Endabschnitts der Aufbissanordnung 100. Zudem kann der Querstrich des "T" des T-förmigen Endabschnitts jeweils auf die Kameras 110 zulaufend - bei einer Ansicht von vorne auf die Beleuchtungskomponente 120 - (leicht) nach oben oder unten gebogen sein und somit - ebenfalls bei einer Ansicht von vorne auf die Beleuchtungskomponente 120 - eine U-Form oder Hufeiesen-Form oder Parabelform bilden. Bei einer solchen Ansicht kann sich am Anfang und Ende des U-förmigen Abschnitts jeweils eine der Kameras 110 befinden. Generell ist die in Figur 3 gezeigte T-Form als beispielhaft zu verstehen. Zusätzlich oder alternativ kann eine U- oder Parabel- oder V-Form vorgesehen sein. Zusätzlich oder alternativ kann die jeweils gewählte Form an den jeweiligen Kieferbogen des Patienten angepasst werden kann. Die Kameras können an dem jeweiligen Ende positioniert sein, können jedoch auch an anderen Stellen, beispielsweise auf dem Bogen des Us, einer U-Form oder den Schenkeln einer U-Form oder V-Form, platziert sein. Ferner können die Kameras beispielsweise so angeordnet sein, dass sie in eine der Richtung des Kieferbogens entgegengesetzte Richtung nach innen blicken. Auf diese Weise kann beispielsweise eine rechte Kamera eine linke Seite abbilden und umgekehrt.

Die Aufbissvorrichtung 10 weist ferner in dem Beispiel aus Figur 3 beispielhaft eine einzige Beleuchtungskomponente 120 auf, um zu veranschaulichen, dass mindestens eine Beleuchtungskomponente 120 vorgesehen sein kann. Die Beleuchtungskomponente 120 ist an der Aufbissanordnung 100 getrennt von den beiden Kameras 110 angeordnet. Die Beleuchtungskomponente 120 ist, bei einem T-förmigen Endabschnitt, beispielhaft in einem Mittelbereich oder in der Mitte des Querstrichs des "T" angeordnet. Bei einem U-förmigen oder hufeisen-förmigen oder parabelförmigen Endabschnitt ist die Beleuchtungskomponente 120 beispielhaft an einem Scheitelpunkt des Endabschnitts angeordnet. Jedenfalls ist die Beleuchtungskomponente 120 derart ausgebildet und an der Aufbissanordnung 100 angeordnet, dass sie zumindest einen Teil der Mundhöhle des Patienten ausleuchten kann.

Hierzu befindet sich die Beleuchtungskomponente 120 in einem Bereich der Aufbissanordnung 100, der bei Aufnahme der Aufbissvorrichtung 10 zwischen den Zähnen des Patienten bei fachgerechter Anwendung in die Mundhöhle des Patienten ragt. Die Beleuchtungskomponente 120 ist in dem Beispiel aus Figur 3, wie beschrieben, an einem Endabschnitt der Aufbissanordnung 100 ausgebildet. Im Gegensatz zu dem Beispiel aus Figur 2 befindet sich die Beleuchtungskomponente 120 nicht an der Position der Kameras 110, genauer gesagt nicht scheibenförmig oder kreisringförmig um die Kameras 110. Hingegen steht die Beleuchtungskomponente 120 von dem beispielhaft T-förmigen oder U-förmigen Endabschnitt in Richtung der Mundhöhle vor. Dadurch kann die Beleuchtungskomponente 120 die Mundhöhle zumindest teilweise ausleuchten. Bei Kombination der Ausführungsbeispiele aus Figur 2 und 3 können mehrere Beleuchtungskomponenten 120 vorgesehen sein, beispielsweise zwei an den in Figur 2 gezeigten Positionen um die Kameras 110 und eine an der in Figur 3 gezeigten Position.

In dem Beispiel aus Figur 3 sind die Kameras 110 und die Beleuchtungskomponente 120 zwar nicht an einer gleichen Fläche, z. B. einer Endfläche oder Grundfläche, der Aufbissanordnung 100 angeordnet. Dennoch wird gewährleistet, dass die Beleuchtungskomponente 120 einen Teil der Mundhöhle ausleuchtet und Blickwinkel der Kameras 110 zumindest teilweise auf den ausgeleuchteten Teil gerichtet sind. Auf diese Weise kann effizient der durch die Kameras 110 aufzunehmende Bereich der Mundhöhle durch die Beleuchtungskomponente 120 ausgeleuchtet werden.

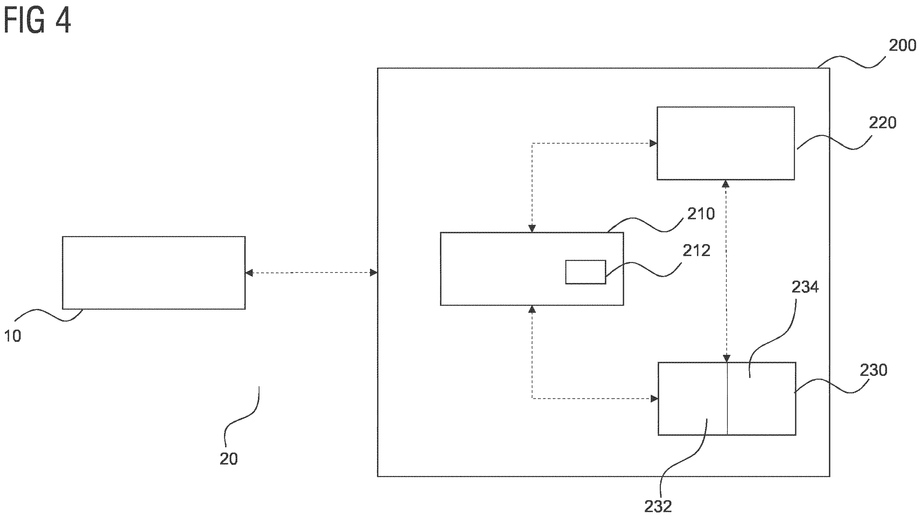

Figur 4 zeigt ein Blockdiagramm eines medizinischen bildgebenden Geräts 20, insbesondere eines dentalen Röntgengeräts. Das medizinische bildgebende Gerät 20 weist eine Aufbissvorrichtung 10 auf. Die Aufbissvorrichtung 10 kann wie in Bezug auf Figuren 2 und 3 beispielhaft beschrieben ausgebildet sein. Demgemäß weist die Aufbissvorrichtung 10 eine Aufbissanordnung 100 auf, die insbesondere eine Aufbissschiene aufweisen oder als solche ausgebildet sein kann. Die Aufbissanordnung 100 ist ausgebildet, zwischen zumindest einem Zahn eines Oberkiefers und zumindest einem Zahn eines Unterkiefers eines Patienten derart aufgenommen zu werden, dass zumindest ein Abschnitt der Aufbissanordnung 100 in eine Mundhöhle des Patienten hineinragt. Die Aufbissanordnung 100 weist mindestens eine Kamera 110 auf, die derart ausgebildet und an der Aufbissanordnung 100 angeordnet ist, mindestens eine Bildaufnahme (optische Aufnahme) zumindest eines Teils der Mundhöhle des Patienten aufzunehmen oder zu erstellen.

Das medizinische bildgebende Gerät 20 weist beispielhaft ein Röntgengerät 200 auf. Das Röntgengerät 200 kann beispielsweise als Panorama-Röntgengerät ausgebildet sein oder ein Panorama-Röntgengerät aufweisen. Das medizinische bildgebende Gerät 20 und insbesondere das Röntgengerät 200 weist eine Röntgenaufnahmevorrichtung 210 auf. Die Röntgenaufnahmevorrichtung 210 ist ausgebildet, mindestens eine Röntgenaufnahme eines abzubildenden Bereichs der Mundhöhle des Patienten unter Berücksichtigung von Informationen über die Bildaufnahme zumindest des Teils der Mundhöhle des Patienten durchzuführen. Die Röntgenaufnahmevorrichtung 210 weist beispielhaft eine DVT-Vorrichtung 212 auf. Die DVT-Vorrichtung 212 ist ausgebildet, zur Aufnahme mindestens einer Röntgenaufnahme, mindestens eine Volumenaufnahme, insbesondere mindestens eine DVT-Aufnahme, des abzubildenden Bereichs der Mundhöhle des Patienten aufzunehmen.

Das medizinische bildgebende Gerät 20 und insbesondere das Röntgengerät 200 weist beispielhaft eine Recheneinheit 220 auf. Die Recheneinheit 220 steht zum Austausch notwendiger Informationen mit der Röntgenaufnahmevorrichtung 210 drahtlos und/oder drahtgebunden in Verbindung. Die Recheneinheit 220 ist ausgebildet, den abzubildenden Bereich der Mundhöhle des Patienten unter Berücksichtigung von Informationen über die Bildaufnahme zumindest eines, insbesondere des, Teils der Mundhöhle des Patienten zu ermitteln. Die Recheneinheit 220 kann den abzubildenden Bereich der Mundhöhle des Patienten beispielsweise automatisch unter Berücksichtigung von Informationen über die Bildaufnahme zumindest eines, insbesondere des, Teils der Mundhöhle des Patienten ermitteln.

Zusätzlich oder alternativ kann die Recheneinheit 220 Informationen über eine Bahnkurve des medizinischen bildgebenden Geräts 20 basierend auf den Informationen über die Bildaufnahme zumindest eines, insbesondere des, Teils der Mundhöhle des Patienten ermitteln. Die Recheneinheit 220 kann beispielsweise die Informationen über eine Bahnkurve des medizinischen bildgebenden Geräts 20 basierend auf der Bildaufnahme zumindest eines, insbesondere des, Teils der Mundhöhle des Patienten ermitteln. Die Informationen über die Bahnkurve können beispielsweise ein Rotationszentrum oder mehrere Rotationszentren der Bahnkurve des medizinischen bildgebenden Geräts 20 aufweisen.

Die Aufbissvorrichtung 10, z. B. die Aufbissanordnung 100 und/oder die mindestens eine Kamera 110, kann drahtlos und/oder drahtgebunden mit dem Röntgengerät 200 und somit mit der Recheneinheit 220 verbunden oder verbindbar sein, d. h. die Verbindung zwischen der Aufbissvorrichtung 10, z. B. der Aufbissanordnung 100 und/oder der mindestens einen Kamera 110, und dem Röntgengerät 200 und somit der Recheneinheit 220 kann eine drahtgebundene Verbindung, eine drahtlose Verbindung oder eine Kombination aus beiden aufweisen. Über die drahtlose und/oder drahtgebundene Verbindung kann die Aufbissvorrichtung 10, z.B. die Aufbissanordnung 100 und/oder die mindestens eine Kamera 110, die von der mindestens einen Kamera 110 aufgenommene mindestens eine Bildaufnahme insbesondere vor Durchführung einer Röntgenaufnahme an das Röntgengerät und somit an die Recheneinheit 220 übermitteln. Die mindestens eine Kamera 110 kann zudem mindestens eine Zwischenbildaufnahme zumindest des Teils der Mundhöhle des Patienten während einer Durchführung der Röntgenaufnahme des abzubildenden Bereichs der Mundhöhle des Patienten aufnehmen (erstellen) und an das Röntgengerät 200 und somit die Recheneinheit 200 übermitteln. Auf diese Weise kann das Röntgengerät 200, insbesondere die Recheneinheit 220, Informationen über eine mögliche Patientenbewegung ermitteln und bei einer Röntgenaufnahme berücksichtigen.

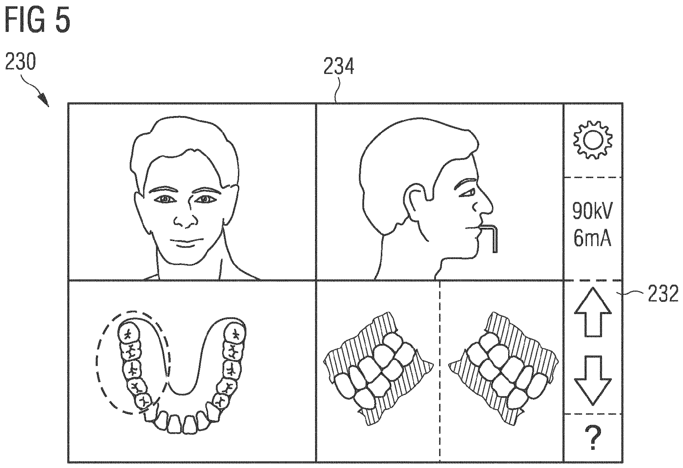

Das medizinische bildgebende Gerät 20 und insbesondere das Röntgengerät 200 weist beispielhaft eine Eingabeeinheit 232 und eine Ausgabeeinheit 234 auf, die beispielhaft in Figur 4 als eine gemeinsame Eingabe-Ausgabeeinheit 230 ausgebildet sind. Dies ist als rein beispielhaft zu verstehen und die Eingabeeinheit 232 und die Ausgabeeinheit 234 können getrennt voneinander in dem Röntgengerät 200 realisiert sein. Die Ausgabeeinheit 234 ist ausgebildet, die Bildaufnahme zumindest des Teils der Mundhöhle auszugeben. Insbesondere kann die Ausgabeeinheit 234 eine Darstellung einer Überlagerung der Bildaufnahme zumindest des Teils der Mundhöhle und des abzubildenden Bereichs der Mundhöhle des Patienten ausgeben. Beispielhafte Details der Ausgabeeinheit 234 sind in Figur 5 dargestellt und werden in Bezug auf Figur 5 genauer beschrieben.

Die Eingabeeinheit 232 ist ausgebildet, eine Eingabe oder Auswahl einer vordefinierten Volumengröße für den abzubildenden Bereich zu erhalten. Zusätzlich oder alternativ ist die Eingabeeinheit 232 ausgebildet, eine Markierung oder Auswahl des abzubildenden Bereichs auf der Bildaufnahme zumindest des Teils der Mundhöhle des Patienten zu erhalten. Die Eingabeeinheit 232 weist, bei einer Ausbildung als gemeinsame Eingabe-Ausgabeeinheit 230, als Eingabeelemente eine oder mehrere Tasten und/oder Touchscreen-Elemente auf der Ausgabeeinheit 234 auf. Die Eingabeeinheit 232 kann eine Eingabe oder Auswahl einer vordefinierten Volumengröße für den abzubildenden Bereich über die Eingabeelemente erhalten. Zusätzlich oder alternativ kann die Eingabeeinheit 232 eine Markierung oder Auswahl des abzubildenden Bereichs auf der Bildaufnahme zumindest des Teils der Mundhöhle des Patienten erhalten. Der abzubildende Bereich der Mundhöhle des Patienten kann über die Eingabeeinheit 232 auf der Bildaufnahme zumindest des Teils der Mundhöhle durch einen Benutzer ausgewählt werden.

In Figur 5 ist beispielhaft eine Eingabe-Ausgabeeinheit 230 gezeigt, die in dem medizinischen bildgebenden Gerät 20 aus Figur 4 eingesetzt werden kann. Auf der rechten Seite der Eingabe-Ausgabeeinheit 230 ist eine Eingabeeinheit 232 mit mehreren Eingabefeldern oder Eingabetasten als Eingabeelemente vorgesehen. Über die Eingabeeinheit 232 können Einstellungen vorgenommen werden. Ferner können Röntgenparameter der Röntgengeräts 200 eingestellt werden. Zudem kann eine Höhenverstellung des medizinischen bildgebenden Geräts 20 vorgenommen werden. Zudem ist eine Hilfefunktion vorgesehen. Die genannten Beispiele können beliebig ergänzt oder ersetzt werden, um die Eingabeeinheit 232 auf die gewünschten Bedürfnisse anzupassen.

Links neben der Eingabeeinheit 232 ist eine Ausgabeeinheit 234 an der Eingabe-Ausgabeeinheit 230 vorgesehen. Die Ausgabeeinheit 234 weist beispielhaft mehrere Ausgabefelder auf, in denen verschiedene Informationen ausgegeben werden können. Beispielsweise können auf einem oder mehreren Ausgabefeldern Kameraaufnahmen von extraoralen Kameras angezeigt werden. Zusätzlich können auf einem Ausgabefeld Bildaufnahmen von den beispielsweise zwei Kameras 110 aus Figur 3 ausgegeben werden. Auf den ausgegebenen Bildaufnahmen kann ein abzubildender Bereich ausgewählt oder bestimmt werden. Der abzubildende Bereich kann als ausgewähltes Volumen auf einem Zahnschema in einem anderen Ausgabefeld der Ausgabeeinheit 234 ausgegeben und ggf. angepasst werden.

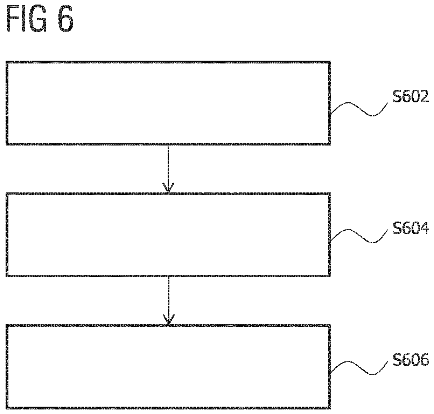

In Figur 6 wird ein Flussdiagramm eines Verfahrens zur Röntgenaufnahme eines abzubildenden Bereichs einer Mundhöhle eines Patienten dargestellt. Das Verfahren umfasst ein derartiges Aufnehmen (S602) einer Aufbissanordnung zwischen zumindest einem Zahn eines Oberkiefers und zumindest einem Zahn eines Unterkiefers eines Patienten, dass zumindest ein Endabschnitt der Aufbissanordnung in eine Mundhöhle eines Patienten hineinragt. Das Verfahren umfasst ein Durchführen (S604) mindestens einer Bildaufnahme zumindest eines Teils der Mundhöhle des Patienten mit mindestens einer an der Aufbissanordnung angeordneten Kamera. Das Verfahren umfasst ein Durchführen (S606) einer Röntgenaufnahme, mit einer Röntgenaufnahmevorrichtung, mindestens eines abzubildenden Bereichs der Mundhöhle des Patienten unter Berücksichtigung von Informationen über die Bildaufnahme zumindest eines, insbesondere des, Teils der Mundhöhle des Patienten.

Weitere Details der in Bezug auf die Figuren 1 bis 6 beschriebenen Vorrichtungen und Verfahren, nämlich der Aufbissvorrichtung 10 aus Figuren 2 und 3, dem medizinischen Gerät 20 aus Figur 4 und dem Verfahren aus Figur 6, werden nun beschrieben.

Durch eine an oder in einem Aufbiss / der Aufbissvorrichtung 10 platzierte Kamera 110 wird ein Bild eines Mund-Innenraumes / einer Mundhöhle während einer Positionierung eines Patienten angezeigt. Nach der Wahl eines vorgegebenen Volumens werden die von diesem Volumen abgebildeten Zähne entsprechend gekennzeichnet, beispielsweise auf dem aufgenommenen Bild eingefärbt. Alternativ kann der Bediener des medizinischen Gerätes 20 auf dem Bild des Mundinnenraumes / der Mundhöhle eine Reihe von Zähnen auswählen. Durch entsprechende Verfahren wird berechnet mit welcher Bahnkurve die entsprechenden Eckpunkte auf dem Sensor / Detektor des medizinischen Geräts 20 abgebildet werden, so dass man eine an den abzubildenden Bereich angepasste Aufnahme durchführen kann.

Durch die Positionierung einer oder mehrerer Kameras 110 sowie einer oder mehrerer Beleuchtungshilfsmittel / Beleuchtungskomponenten 120 an oder in dem Aufbiss / der Aufbissvorrichtung 10 wird es ermöglicht, eine Abbildung der Mundhöhle parallel zu weiteren Positionierungs-Hilfen darzustellen. Bei der Auswahl einer vordefinierten Volumengröße kann, aufgrund der bekannten Referenzposition des Aufbisses / der Aufbissvorrichtung 10, das Bild der Mundhöhle sowie ggfs. mindestens ein Bild der extraoralen Kamera, mit dem abzubildenden Bereich überlagert werden, so dass der Bediener des medizinischen Geräts 20 nicht nur auf der Außenseite des Patienten sondern auch in einem dentalen Bereich direkt erkennt, welche Region abgebildet werden wird.

Alternativ ist es möglich, durch entsprechende Markierung auf dem Kamerabild, den abzubildenden Bereich auszuwählen. Durch die bekannte Referenzposition des Aufbisses, in Kombination mit z.B. einer Weitenmessung über die Schläfenstützen oder anderer Hilfsmittel, kann der abzubildende Bereich in einem großformatigen, bekannten Volumen festgestellt werden und über entsprechende algorithmische Verfahren die entsprechende Position des Bildes auf dem Detektor des medizinischen Geräts 20 errechnet werden. Mittels Verfahren der Blende des medizinischen Geräts 20 wird dann nur der ausgewählte Bereich des Mundraumes / der Mundhöhle mit dem medizinischen Gerät (im Unterschied zu den Kameras) aufgenommen und in der DVT Aufnahme abgebildet. Alternativ kann durch die entsprechende freie Wahl des Rotationszentrums eine für den abzubildenden Bereich passende Bahnkurve ausgewählt werden und die Aufnahme dementsprechend getätigt werden.

Ein weiterer Vorteil der Aufbisskamera 110 ist es, während der Durchführung der Aufnahme eine optische Aufnahme der Mundhöhle zu generieren. Bedingt durch vorhandene Marker (z.B. Zahnzwischenräume) oder andere vorbekannte Verfahren kann man somit Informationen über eine mögliche Patientenbewegung während der Aufnahme erhalten und diese zur Korrektur von Bewegungsartefakten heranziehen.

Durch die zusätzliche Information aus der Mundhöhle wird es dem Bediener ermöglicht, den Patienten zuverlässig zu positionieren und eine an die Fragestellung angepasste DVT und/oder Panorama -Aufnahme durchzuführen und somit die Dosis für den Patienten zu minimieren.

## Patentansprüche

1. Vorrichtung aufweisend:
eine Anordnung, wobei die Anordnung derart anordenbar oder aufnehmbar ist, dass zumindest ein Endabschnitt der Anordnung in einen Hohlraum hineinragt, wobei zumindest ein Abschnitt der Anordnung druckbeaufschlagt ist, wenn die Anordnung derart angeordnet oder aufgenommen ist, dass zumindest der Endabschnitt der Anordnung in den Hohlraum hineinragt; und
mindestens eine Aufnahmevorrichtung, die derart ausgebildet und an der Anordnung angeordnet ist, mindestens eine Datenaufnahme zumindest eines Teils des Hohlraums durchzuführen.

2. Vorrichtung nach Anspruch 1, wobei die mindestens eine Aufnahmevorrichtung an dem Endabschnitt der Anordnung ausgebildet ist.

3. Vorrichtung nach Anspruch 1 oder 2, wobei zumindest der Endabschnitt der Anordnung nicht druckbeaufschlagt ist, wenn die Anordnung derart angeordnet oder aufgenommen ist, dass zumindest der Endabschnitt der Anordnung in den Hohlraum hineinragt.

4. Vorrichtung nach einem der Ansprüche 1 bis 3, wobei die Vorrichtung ferner aufweist:
mindestens eine ausstrahlende Vorrichtung, die an der Anordnung angeordnet ist.

5. Vorrichtung nach Anspruch 4, wobei die mindestens eine ausstrahlende Vorrichtung an dem Endabschnitt der Anordnung ausgebildet ist.

6. Vorrichtung nach Anspruch 4 oder 5, wobei die mindestens eine ausstrahlende Vorrichtung eine Lichtquelle, eine Röntgenquelle und/oder eine Laserquelle aufweist oder als eine Lichtquelle, eine Röntgenquelle oder eine Laserquelle ausgebildet ist.

7. Vorrichtung nach einem der Ansprüche 4 bis 6, wobei die mindestens eine ausstrahlende Vorrichtung derart ausgebildet und an der Anordnung angeordnet ist, im Zusammenwirken mit der Aufnahmevorrichtung zumindest einen Teil des Hohlraums abzubilden.

8. Vorrichtung nach einem der Ansprüche 1 bis 7, wobei die Vorrichtung ausgebildet ist, die mindestens eine Datenaufnahme an ein übergeordnetes System zur weiteren Verwendung weiterzuleiten.
